(19) Europäisches Patentamt
European Patent Office
Office européen des brevets

(11) Veröffentlichungsnummer: **0 083 010**
**B1**

(12) # EUROPÄISCHE PATENTSCHRIFT

(45) Veröffentlichungstag der Patentschrift: **04.12.85**

(21) Anmeldenummer: **82111519.3**

(22) Anmeldetag: **11.12.82**

(51) Int. Cl.⁴: **C 07 C 43/205**, C 07 C 43/23, C 07 D 213/64, C 07 C 41/16, C 07 C 41/26

(54) **Diether von m- oder p-Hydroxyphenolen und Verfahren zur Herstellung der Diether oder Monoether von m- oder p-Hydroxyphenolen.**

(30) Priorität: **28.12.81 DE 3151589**

(43) Veröffentlichungstag der Anmeldung: **06.07.83 Patentblatt 83/27**

(45) Bekanntmachung des Hinweises auf die Patenterteilung: **04.12.85 Patentblatt 85/49**

(84) Benannte Vertragsstaaten: **BE CH DE FR GB IT LI NL**

(56) Entgegenhaltungen:
**EP - A - 0 017 767**
**FR - A - 2 288 089**

**ARKIV FÖR KEMI, Band 17, Nr. 38, 8. Februar 1961, Seiten 393-400, SVEN-OLOV LAWESSON et al.: "Studies on peroxy compounds. XII. Preparation and dealkylation of t-butyl aryl ethers"**
**ARKIV FÖR KEMI, Band 26, Nr. 18, 25. Mai 1966, Seiten 199-211 H.J. JAKOBSEN et al.: "Mass spectra of tert-butyl ethers"**
**CHEMICAL ABSTRACTS, Band 93, Nr. 3, 21. Juli 1980, Seite 665, Nr. 26034j, Columbus, Ohio, USA YU. A. OL'DEKOP et al.: "Simple synthesis of the tert-butyl ether of phenol"**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Becker, Rainer, Dr., Sonnenwendstrasse 83, D-6702 Bad Duerkheim (DE)**
Erfinder: **Hoffmann, Werner, Dr., Ringstrasse 11 c, D-6708 Neuhofen (DE)**
Erfinder: **Oeser, Heinz-Guenter, Dr., Mohnstrasse 25, D-6716 Dirmstein (DE)**
Erfinder: **Rohr, Wolfgang, Dr., In der Dreispitz 13, D-6706 Wachenheim (DE)**
Erfinder: **Varwig, Juergen, Dr., Am Goetzenberg 1, D-6900 Heidelberg (DE)**

**EP 0 083 010 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Diether von m- oder p-Hydroxyphenolen und Verfahren zur Herstellung der Diether oder Monoether von m- oder p-Hydroxyphenolen durch Umsetzung von m- oder p-tertiärem Butoxyphenol der Formel II

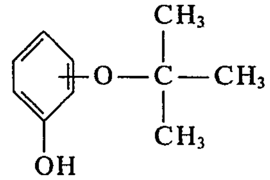

(II)

mit Halogenverbindungen in Gegenwart der Hydroxide, Carbonate und/oder Hydrogencarbonate von Alkalien oder Erdalkalien und von unter den Reaktionsbedingungen inerten, organischen Lösungsmitteln bei einer Temperatur von 20°C bis 150°C und gewünschtenfalls Umsetzung der so gebildeten Diether mit verdünnten wäßrigen anorganischen Säuren bei einer Temperatur von 30 bis 100°C zu den Monoethern.

Die Veretherung nur einer Hydroxygruppe des Hydrochinons bzw. Resorcins mit olefinischen, aromatischen oder heterocyclischen Resten gestaltet sich schwierig und gelingt nur in besonderen Fällen mit besseren Ausbeuten. In der Regel ist hierzu jedoch ein großer Überschuß an Hydrochinon bzw. Resorcin erforderlich, was unwirtschaftlich ist.

Wesentlich einfacher und einheitlicher verläuft die Synthese bei Verwendung einer Schutzgruppe (europ. Patentanmeldung 0 017 767). Man geht zum Beispiel von Hydrochinon-mono-methylether bzw. -benzylether aus, verethert diese zunächst an der noch freien Hydroxygruppe mit olefinischen, aromatischen oder heterocyclischen Resten und spaltet dann anschließend die Schutzgruppe wieder ab. Die Schwierigkeiten treten bei dieser Synthesefolge jedoch in der spezifischen Abspaltung der Schutzgruppe auf. Wie eigene Versuche zeigten, gelingt die Entfernung des Methylrestes nur unter recht drastischen Bedingungen, z. B. mit Essigsäure/Bromwasserstoffsäure unter Rückfluß und mit Reaktionszeiten von mehreren Tagen. Auch eine Spaltung der zweiten Etherbindung tritt ein, so daß solche Verfahren unwirtschaftlich sind.

Die Abspaltung der Benzylschutzgruppe geschieht durch katalytische Hydrierung mit Palladium auf Aktivkohle (JP-OS 55 160–762). Auch hierbei können Folgereaktionen eintreten. Zudem ist diese Methodik aufwendig und somit unwirtschaftlich.

Es wird in DE-OS 2 546 251 beschrieben, daß man substituierte Halogenpyridine mit p-(C 1–C 5)-Alkoxyphenolen zu den entsprechenden Pyridyl-Alkoxyphenylethern in Gegenwart eines alkalischen Materials umsetzen und diese durch Dealkylierung in die entsprechenden Pyridyl-p-hydroxyphenylether überführen kann. Ausführungsbeispiele bzw. nähere Angaben bezüglich der Dealkylierung mit der C 1–C 5-Gruppe fehlen. Insbesondere wird die tertiäre Butylgruppe weder beschrieben, noch werden die Herstellung und Dealkylierung solcher tertiärer Butoxy-Verbindungen näher angegeben bzw. mit einem Beispiel gezeigt. Als allgemeine Form der Dealkylierung von $C_1$–$C_5$)-Alkoxygruppen wird lediglich eine Umsetzung a) bei bevorzugt 150–200°C unter Verwendung von Pyridinhydrochlorid als Dealkylierungsmittel, oder b) bei einer Temperatur nahe des Siedepunktes einer niedrigen Fettsäure als verwendeten Lösungsmittel unter Verwendung einer Halogenwasserstoffsäure als Dealkylierungsmittel genannt. Als Fettsäure werden Essigsäure und Essigsäureanhydrid, als Halogenwasserstoffsäure Brom- und Jodwasserstoffsäure angegeben.

In den folgenden Literaturstellen

(1) ARKIV FÖR KEMI, Band 17, Nr. 38, 8. 2. 1961, Seiten 393 bis 400,
(2) ARKIV FÖR KEMI, Band 26, Nr. 18, 25. 5. 1966, Seiten 199 bis 211,
(3) CHEMICAL ABSTRACTS, Band 93, Nr. 3, 21. 7. 1980, Seite 665, Nr. 26034j

wird die Herstellung von t-Butylphenylethern und/oder die Abspaltung der t-Butylgruppe aus diesen Verbindungen beschrieben.

(1) lehrt (S. 395, letzter Absatz), daß t-Butylphenylether und entsprechende o-Tolyl-, m-Tolyl-, p-Bromphenyl-t-butylether stabile Verbindungen sind und selbst bei längerem (23 bis 24 h) Erhitzen und hohen Dealkylierungstemperaturen (200°C) nicht dealkyliert werden. Lediglich bei 2 Verbindungen, nämlich p-Tolyl- und dem p-Anisylether, konnte bei so hohen Temperaturen eine Dealkylierung erzielt werden (S. 395 unten bis S. 396, Zeile 2). Der Fachmann konnte diesen Befund aus (1) nicht auf das Verhalten von anderen Diethern ausdehnen, denn es wurde lediglich das Verhalten einer einzigen Verbindung, nämlich des p-Methoxy-diethers, beschrieben; es war daher überraschend, daß die erfindungsgemäßen, strukturell sehr verschiedenen Diether der Formel Ia in der Stufe b) sehr gute Ausbeuten an Monoethern der Formel Ib liefern.

Gerade (1) lehrt, daß das Verhalten auch analoger Stoffe bei der Hitzebehandlung nicht vorhergesagt werden kann: t-Butyl-p-tolylether kann quantitativ zum p-Kresol dealkyliert werden (S. 399, 3. Absatz);

jedoch liefert der t-Butyl-m-tolylether während 11 Stunden bei 200°C kein m-Kresol (S. 399, 2. Absatz). Entsprechend (S. 398, letzter Absatz) ist auch der o-Tolylether stabil. Mit anderen Worten, der kleine Strukturunterschied in der Stellung(p- gegenüber m- und o-Stellung) liefert bei derselben thermischen Behandlung bei sonst gleicher Struktur des Ausgangsstoffs gegensätzliche Ergebnisse. (1) lehrt aber darüber hinaus, daß auch dieser Strukturunterschied nicht verallgemeinert werden kann; bei gleicher Stellung (p-) und unterschiedlichem p-Substituenten (Brom bzw. Methyl bzw. Methoxy) erhält man ebenfalls unvorhersehbar gegensätzliche (p-Bromphenylether stabil, p-Methylphenylether quantitativ umsetzbar) Ergebnisse (S. 395, letzter Absatz). Es war daher überraschend, daß entgegen der Lehre von (1) trotz großer Strukturunterschiede der Substituenten die erfindungsgemäßen Diether mit sehr guten Ausbeuten an Phenolen dealkyliert werden.

Hinzu kommt, daß (1) nur einen Diether, nämlich den p-Anisylether (S. 399, 4. Absatz), aber seine von der erfindungsgemäßen stets unterschiedlichen Arbeitsweisen an mehreren Beispielen erläutert. Für seine thermische Behandlung verwendet (1) Temperaturen von 150 bis 200°C, keine Lösungsmittel und bei den stabilen Ethern lange Reaktionszeiten (11—24 h). Es war daher überraschend, daß die erfindungsgemäßen, unter sich dazu noch unterschiedlichen Diether Ia gerade mit Lösungsmitteln, und zwar in der Kombination von Wasser und Alkanolen, und darüber hinaus bei weit tieferen Temperaturen (Stufe b) 30 bis 100°C) die sehr guten Ergebnisse liefert. Auch konnte aus der Angabe eines einzigen sauren Dealkylierungskatalysators, nämlich p-Toluolsulfonsäure, nicht erwartet werden, daß gerade Wasser/Alkohol/anorganische Säure-Gemische und bei den erfindungsgemäßen tieferen Temperaturen strukturell von dem bekannten sehr unterschiedliche Diether vorteilhafte Ergebnisse liefern.

(2) zeigt lediglich die Abspaltung der t-Butylgruppe bei Methoxyphenyl- und t-Butoxyphenyl-t-Butylethern. (3) beschreibt die Herstellung des t-Butylphenylethers und seiner m-Hydroxy- bzw. m-t-Butoxy-Derivate. Das erfindungsgemäße Verfahren ist daher auch im Hinblick auf (2) und (3) aus den zu (1) genannten Gründen überraschend.

Es wurde nun gefunden, daß man Diether von m- oder p-Hydroxyphenolen der Formel

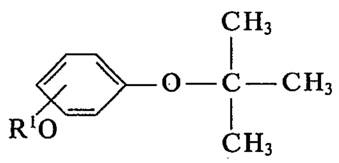

(Ia)

oder Monoether von m- oder p-Hydroxyphenolen der Formel

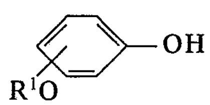

(Ib)

worin die beiden Hydroxygruppen in m- oder p-Stellung zueinander stehen und $R^1$ einen aliphatischen Rest oder den Rest

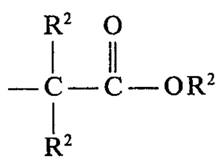

den Rest

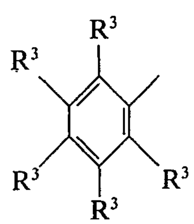

den Rest

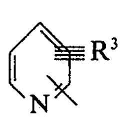

den Rest

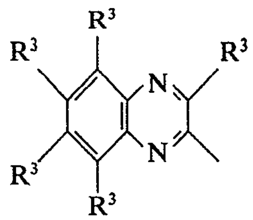

oder den Rest

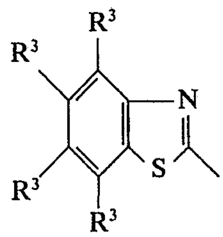

bedeutet, die einzelnen Reste $R^2$ und $R^3$ gleich oder verschieden sein können und jeweils ein Wasserstoffatom, einen aliphatischen, araliphatischen oder aromatischen Rest bezeichnen, $R^3$ auch jeweils für ein Halogenatom, eine Nitrogruppe, Cyangruppe oder eine Alkoxygruppe steht, durch Umsetzung eines Monoethers von Dihydroxyverbindungen mit Halogenverbindungen unter Bildung eines Diethers und gewünschtenfalls Abspaltung der ursprünglichen Monoethergruppe, vorteilhaft erhält, wenn man

a) m- oder p-tertiäres Butoxyphenol der Formel

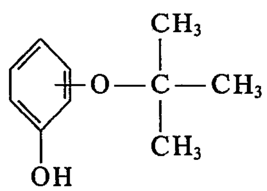

(II)

mit Halogenverbindungen der Formel

$R^1$–X (III)

worin $R^1$ die vorgenannte Bedeutung besitzt und X ein Halogenatom bedeutet, in Gegenwart der Hydroxide, Carbonate und/oder Hydrogencarbonate von Alkalien oder Erdalkalien und von unter den Reaktionsbedingungen inerten, organischen Lösungsmitteln bei einer Temperatur von 20 bis 150°C zu den Endstoffen Ia umsetzt und gegebenenfalls

b) die so erhaltenen Endstoffe Ia mit einem Gemisch aus Wasser, wassermischbaren Alkanolen und anorganischen Säuren bei einer Temperatur von 30 bis 100°C zu den Endstoffen Ib umsetzt.

Weiterhin wurden die neuen Diether von m- oder p-Hydroxyphenol der Formel

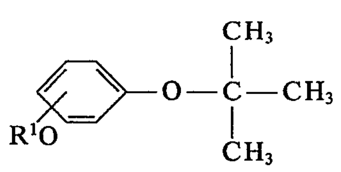

(Ia)

worin die beiden Ethergruppen in m- oder p-Stellung zueinander stehen und $R^1$ einen aliphatischen Rest oder den Rest

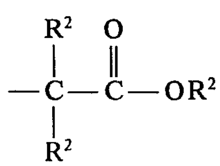

oder den Rest

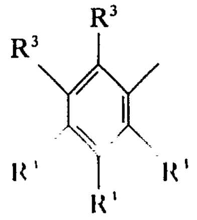

oder den Rest

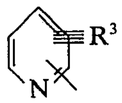

oder den Rest

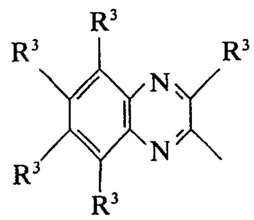

oder den Rest

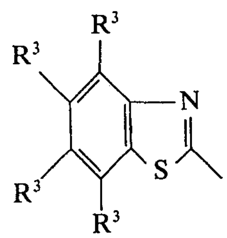

bedeutet, die einzelnen Reste $R^2$ und $R^3$ gleich oder verschieden sein können und jeweils ein Wasserstoffatom, einen aliphatischen, araliphatischen oder aromatischen Rest bezeichnen, $R^3$ auch jeweils für ein Halogenatom, eine Nitrogruppe, eine Cyangruppe oder eine Alkoxygruppe steht, gefunden.

Die Umsetzung kann im Falle der Verwendung von 2-Chlor-5-nitropyridin, p-(tert.-Butoxy)-phenol und Natronlauge durch die folgenden Formeln wiedergegeben werden:

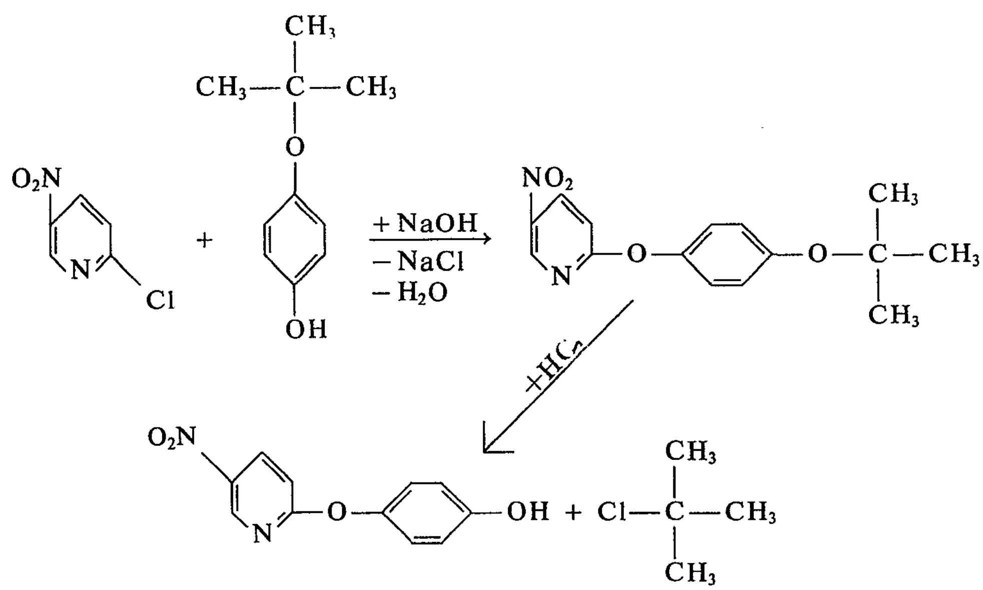


Im Hinblick auf den Stand der Technik liefert das erfindungsgemäße Verfahren auf einfachem und wirtschaftlichem Wege Diether oder Monoether von p- und m-Hydroxyphenolen in guter Ausbeute und Reinheit. Gleichzeitig werden die neuen Diether mit einer tert.-Butoxygruppe als Etherkomponente

durch Ausführungsbeispiele charakterisiert. Es war bisher nicht bekannt, daß gerade die tert.-Butylgruppe überraschend bei den neuen Diethern Ia schneller und/oder bei tieferer Temperatur abgespalten wird als andere Alkylgruppen, wie die Methylgruppe. Das Gesamtverfahren (Stufen a) und b)) ist so im Hinblick auf die DE-OS 2 546 251 einfacher, wirtschaftlicher und liefert eine höhere Raum-Zeit-Ausbeute. Es ist überraschend, daß die erfindungsgemäßen Bedingungen in Stufe a) und noch mehr in Stufe b) nicht allein bei Pyridylethern, sondern vorteilhaft auch bei anderen, von der Pyridylgruppe stark unterschiedlichen Etherkomponenten gute Ergebnisse liefern. Es war auch nicht zu erwarten, daß gerade die erfindungsgemäßen Bedingungen der Stufe b), z. B. die Verwendung von Alkanol und Wasser, mit den neuen Endstoffen Ia eine vorteilhafte Dealkylierung erlauben. Ebenfalls ist es überraschend, daß die Dealkylierung im Hinblick auf DE-OS 2 546 251 nicht bei einer Temperatur nahe des Siedepunktes des Lösungsmittels erfolgen muß und die vorteilhaften erfindungsgemäßen Ergebnisse sich auch ohne Zusatz niedriger Fettsäuren, z. B. Essigsäure, einstellen. Die milderen Reaktionsbedingungen erlauben gleichzeitig die Herstellung auch empfindlicher Endstoffe, z. B. von Fluoralkoxy-Verbindungen.

Die Ausgangsstoffe II und III können in stöchiometrischer Menge oder im Überschuß jeder Komponenten bezogen auf die andere, zweckmäßig in einem Verhältnis von 1 bis 10, vorzugsweise 1 bis 2 Mol Ausgangsstoff III je Mol Ausgangsstoff II umgesetzt werden. Bevorzugte Ausgangsstoffe II, III und dementsprechend bevorzugte Endstoffe Ia und Ib sind solche, in deren Formeln die beiden Hydroxigruppen in m- oder p-Stellung zueinander stehen und $R^1$ einen Alkylrest mit 1 bis 6 Kohlenstoffatomen, einen Halogenalkylrest, bevorzugt mit 1 bis 6 Kohlenstoffatomen und substituiert mit 1 oder 2 Chloratomen und/oder Fluoratomen oder den Rest

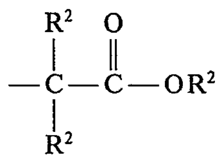

den Rest

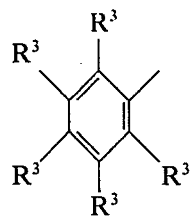

den Rest

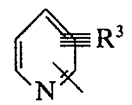

den Rest

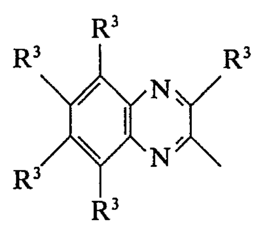

oder den Rest

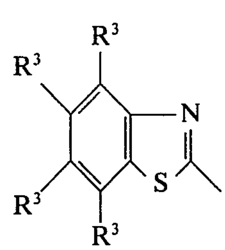

bedeutet, die einzelnen Reste $R^2$ und $R^3$ gleich oder verschieden sein können und jeweils ein Wasserstoffatom, einen Alkylrest mit 1 bis 6 Kohlenstoffatomen, der gegebenenfalls noch durch 1 oder 2 Chloratome und/oder Fluoratome substituiert sein kann, einen Aralkylrest oder Alkylarylrest mit 7 bis 12 Kohlenstoffatomen oder einen Arylrest mit 6 bis 10 Kohlenstoffatomen bezeichnen, $R^3$ auch jeweils für ein Chloratom oder Fluoratom, eine Nitrogruppe, Trifluormethyl-, Trichlormethyl-gruppe, eine Cyangruppe oder eine Alkoxygruppe mit 1 bis 6 Kohlenstoffatomen steht, X ein Chloratom oder Fluoratom bedeutet. Die vorgenannten Reste können noch durch unter den Reaktionsbedingungen inerte Gruppen, z. B. Alkylgruppe oder Alkoxygruppen mit jeweils 1 bis 4 Kohlenstoffatomen, substituiert sein.

Geeignete Ausgangsstoffe III sind beispielsweise: Methyl-, Ethyl-, Propyl-, Isopropyl-, Butyl-, sek.-Butyl-, Isobutyl-, tert.-Butylchlorid oder -fluorid; $\alpha$-Chloressigsäure, $\alpha$-Fluoressigsäure und entsprechende durch die Methyl-, Ethyl-, Propyl-, Isopropyl-, Butyl-, Isobutyl-, sek.-Butyl-, tert.-Butyl-, Phenyl-, Xylyl-, Benzylgruppe einfach oder zweifach in $\alpha$-Stellung substituierte $\alpha$-Chloressigsäure oder $\alpha$-Fluoressigsäure und entsprechende Methyl-, Ethyl-, Propyl-, Isopropyl-, Butyl-, Isobutyl-, sek.-Butyl-, tert.-Butyl-, Benzyl-, Xylyl-, Phenylester; durch Alkyl-, Chloralkyl-, Fluoralkylgruppen mit 1 bis 4 Kohlenstoffatomen und gegebenenfalls 1 oder 2 Halogenatome, insbesondere Chloratome oder Fluoratome, durch Aralkylgruppen mit 7 bis 12 Kohlenstoffatomen oder Arylgruppen mit 6 bis 10 Kohlenstoffatomen, Chloratome, Fluoratome, Nitrogruppen, Cyangruppe oder Alkoxygruppen mit 1 bis 4 Kohlenstoffatomen substituiertes oder unsubstituiertes Phenylchlorid, oder -fluorid, Pyridin-2- oder 4-chlorid bzw. -fluorid, Chinoxalinchlorid oder -fluorid, Benzthiazol-2-chlorid oder -fluorid. Im allgemeinen substituierten an den aromatischen oder heterocyclischen Ringen neben den Halogenatomen 1 bis 2 der vorgenannten Substituenten bzw. sind die Ringe unsubstituiert.

Bevorzugte Ausgangsstoffe III sind Chlordifluormethan, 4-Trifluor- und 4-Trichlormethylphenylchlorid, 4-Nitro-1,2-phenyldichlorid, 2-Chlor-5-jod-phenylchlorid, 4-Nitrophenylchlorid, 2-Chlor-4-nitropyridin, 2,3,5-Trichlorbenzol, 2,6-Dichlorchinoxalin, 2-Chlorbenzthiazol, der Methyl-, Ethyl-, Propyl-, n-Butyl- oder Isobutylester von $\alpha$-Brom- bzw. $\alpha$-Chlorpropionsäure, 2-Nitro-4-trifluormethylchlorbenzol, 2-Brom-5-iodpyridin, 2-Chlor-5-nitropyridin, 2-Brom-3,5-dichlorpyridin, 2,3,5-Trichlorpyridin.

p-(tert.-Butoxy)phenol kann nach bekannten Vorschriften hergestellt werden (J. Chromatogr. 10, 42 bis 67 (1963); C. A. 60, 6773 b). Die m-Verbindung wird analog synthetisiert.

Die Reaktion wird in Stufe a) bei einer Temperatur von 20°C bis 150°C, vorzugsweise 20°C bis 100°C, in Stufe b) bei einer Temperatur von 30 bis 100°C, vorzugsweise 40 bis 100°C, insbesondere 45 bis 80°C, drucklos oder unter Druck, diskontinuierlich oder kontinuierlich durchgeführt. Unter den Hydroxiden, Carbonaten und Hydrogencarbonaten als Katalysatoren sind Natrium, Kalium und Calcium die bevorzugten Kationen. Zweckmäßig ist ein Verhältnis von 1 bis 10, vorzugsweise 1 bis 1,5 Äquivalenten Alkali- bzw. Erdalkaliverbindungen je Mol Ausgangsstoff III.

In Stufe a) verwendet man unter den Reaktionsbedingungen inerte, organische Lösungsmittel. Als Lösungsmittel der Stufe a) kommen z. B. in Frage:

Alkanole und Cycloalkanole wie Ethanol, Methanol, n-Butanol, Isobutanol, tert.-Butanol, Glykol, Glycerin, sek.-Butanol, n-Propanol, Isopropanol, Amylalkohol, Cyclohexanol, 2-Methyl-4-pentanol, Ethylenglykolmonoethylether, 2-Ethylhexanol, Methylglykol, n-Hexanol, Isohexylalkohol, Isoheptylalkohol, n-Heptanol, Ethylbutanol, Nonylalkohol, Dodecylalkohol, Methylcyclohexanol, Diacetonalkohol, insbesondere solche mit 1 bis 6 Kohlenstoffatomen; Schwefelkohlenstoff, Dioxan, Tetrahydrofuran, Sulfoxide wie Dimethylsulfoxid, Diethylsulfoxid, Dimethylsulfon, Diethylsulfon, Methylethylsulfon, Tetramethylensulfon; und entsprechende Gemische. Zweckmäßig verwendet man das Lösungsmittel in einer Menge von 400 bis 10 000 Gewichtsprozent, vorzugsweise von 400 bis 2000 Gewichtsprozent, bezogen auf Ausgangsstoff II.

Die Behandlung des Endstoffes Ia in der Stufe b) wird in Gegenwart von anorganischer Säure, vorteilhaft mit einer Menge von 0,001 bis 1, insbesondere von 0,01 bis 0,1 Äquivalenten Säure, bezogen auf 1 Mol Endstoff Ia, durchgeführt. Anstelle einbasischer Säuren können auch äquivalente Mengen mehrbasischer Säuren zur Anwendung gelangen. Beispielsweise sind folgende Säuren geeignet: Chlorwasserstoff, Bromwasserstoff, Jodwasserstoff, Perchlorsäure, Schwefelsäure, Phosphorsäure, salpetrige Säure, Salpetersäure, Kohlensäure. Bei verdünnten, wäßrigen Säuren sind 1- bis 20gewichtsprozentige Säuren, z. B. 1- bis 20gewichtsprozentige Salzsäure, 1- bis 20gewichtsprozentige Schwefelsäure oder 1- bis 20gewichtsprozentige Phosphorsäure, vorteilhaft. Bevorzugt sind Salzsäure, Phosphorsäure und insbesondere Schwefelsäure.

Als Alkanol wird im allgemeinen ein völlig mit Wasser mischbares Alkanol, zweckmäßig Methanol oder Ethanol, verwendet. Vorteilhaft kommen Mengen von 0,1 bis 1000, insbesondere 0,1 bis 100 Gramm Wasser (eingeschlossen der Wassergehalt der verdünnten Säure) und von 1 bis 10 000, insbesondere 1 bis 100 Gramm Alkanol je Gramm Endstoff Ia in Betracht. Besonders bevorzugt ist eine Kombination von 0,1 bis 1000 Gramm Wasser Alkanol je Gramm Säure, insbesondere Schwefelsäure.

Die Reaktion kann wie folgt durchgeführt werden: Ausgangsstoff II, III, basischer Katalysator und Lösungsmittel werden 0,5 bis 5 Stunden bei der Reaktionstemperatur gehalten. Dann wird Endstoff Ia in üblicher Weise, z. B. durch Wasser- und Säurezugabe bis zur Einstellung von pH 3, Absaugen und Wäsche des ausgeschiedenen Festgutes, isoliert. Gewünschtenfalls wird der Endstoff Ia im Gemisch mit Alkanol, Wasser und anorganischer Säure 0,5 bis 10 Stunden bei der Reaktionstemperatur der Stufe b) gehalten. Der so erhaltene Endstoff Ib wird aus dem Gemisch in üblicher Weise, z. B. Wasserzugabe, Neutralisation mit z. B. Natronlauge, Absäuren und Wäsche, isoliert.

Die nach dem Verfahren der Erfindung herstellbaren Endstoffe Ia und Ib sind wertvolle Ausgangsstoffe für die Herstellung von Farbstoffen, Pharmazeutika und Pflanzenschutzmitteln. So können z. B. aus ihnen die in den DE-OS 2 433 067, 2 223 894, 3 004 770, 2 546 251, und der europäischen Patentanmeldung 0 002 246 beschriebenen Pflanzenschutzmittel hergestellt werden, z. B.

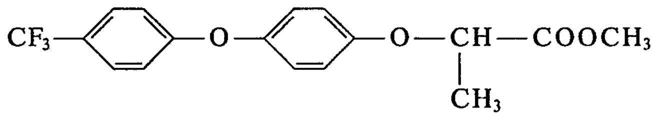

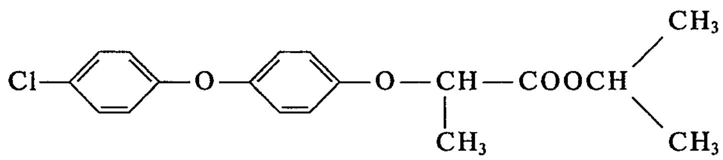

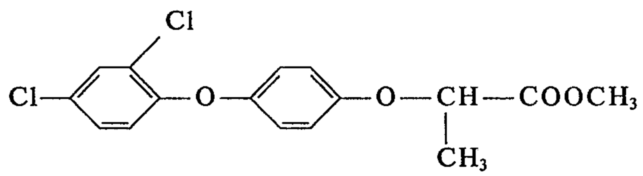

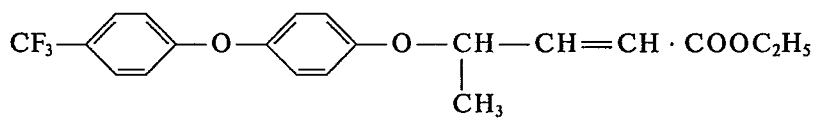

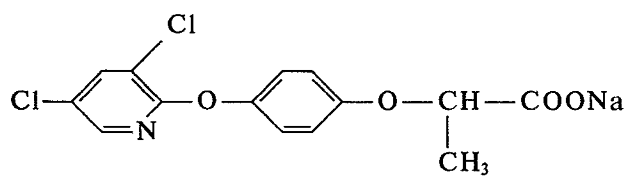

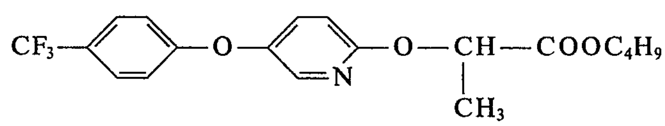

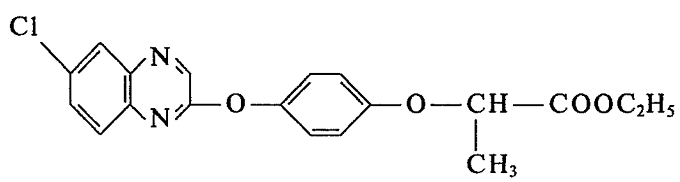

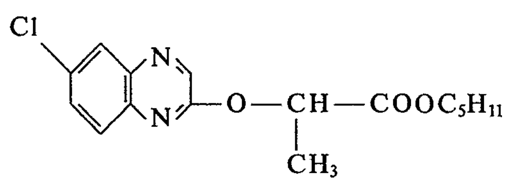

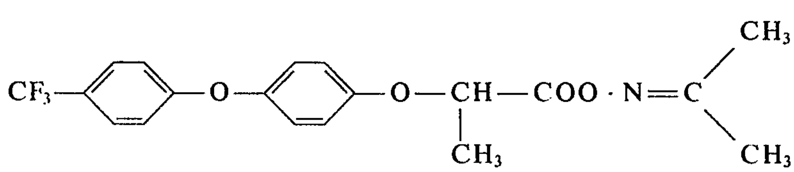

Besonders bevorzugte Endstoffe Ia für die Herstellung von Pflanzenschutzmitteln sind

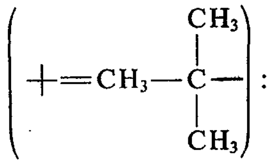

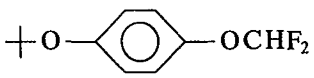

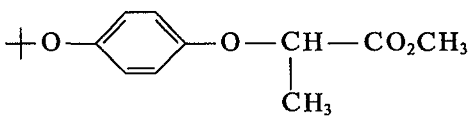

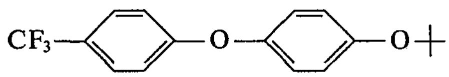

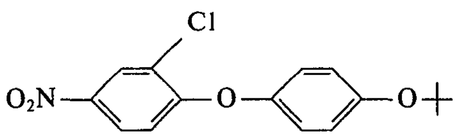

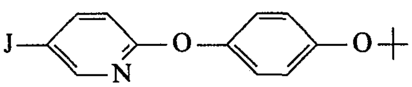

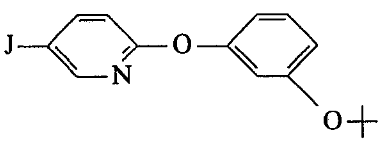

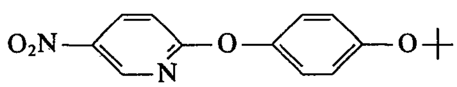

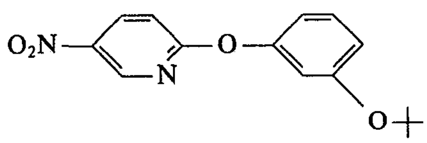

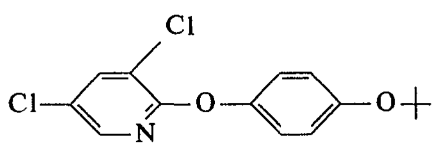

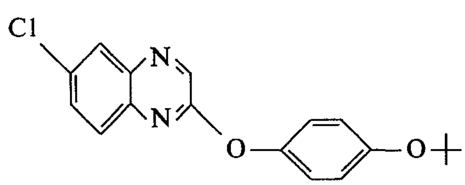

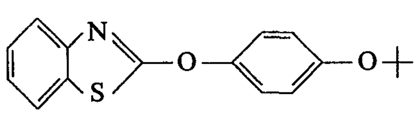

Ebenfalls wird bezüglich der Verwendung auf die Druckschriften zum Stand der Technik verwiesen.

Beispiel 1

a) 32 Gramm 2-Chlor-5-nitropyridin, 33 Gramm p-(tert.-Butoxy)-phenol, 10,5 Gramm Calciumhydroxid und 200 Milliliter Dimethylsulfoxid wurden 5 Stunden bei 80°C gerührt, in Wasser gegossen, mit verdünnter Schwefelsäure auf pH 3 eingestellt, der ausgefallene Niederschlag

abgesaugt, mit Wasser gewaschen und im Vakuum getrocknet. Man erhielt 53 Gramm 4-(5'-Nitropyridyl-2'-oxy)phenyl-(tert.-butyl)ether vom Festpunkt 109 bis 111°C.

b) 13 Gramm des nach Beispiel 1a) erhaltenen 4-(5'-Nitropyridyl-2-oxy)phenyl-(tert.-butyl)ethers, 200 Milliliter Ethanol, 50 Gramm Wasser und 10 Milliliter konz. Schwefelsäure wurden 4 Stunden am Rückfluß (78°C) erwärmt, auf Raumtemperatur abgekühlt, in Wasser gegossen, mit Natronlauge neutral gestellt, der ausgefallene Niederschlag abgesaugt, mit Wasser gewaschen und im Vakuum getrocknet. Man erhielt 8,5 Gramm 4-(5'-Nitropyridyl-2'-oxy)phenol vom Festpunkt 165°C bis 170°C.

## Beispiel 2

a) 33,2 Gramm p-(tert.-Butoxy)phenol, 13 Gramm 85gewichtsprozentiges Kaliumhydroxid und 100 Milliliter Methanol wurden 1,5 Stunden unter Rühren zum Rückfluß (65°C) erwärmt. Anschließend wurde im Vakuum das Lösungsmittel abgezogen, der Rückstand mit 200 Milliliter Dimethylsulfoxid und 50 Gramm 2-Brom-5-jodpyridin versetzt, 6 Stunden bei 120°C gerührt, auf Raumtemperatur abgekühlt, in Wasser gegossen und mit Dichlormethan extrahiert. Die organische Phase wurde mehrmals mit Wasser ausgeschüttelt, das Lösungsmittel im Vakuum abgezogen und der Rückstand aus Petrolether umkristallisiert. Man erhielt 62 Gramm 4-(5'-Jod-pyridyl-2'-oxy)phenyl(tert.-butyl)ether vom Festpunkt 48°C.

b) 30 Gramm des nach Beispiel 2a) erhaltenen 4-(5'-Jod-pyridyl-2'-oxy)phenyl(tert.-butyl)ethers, 100 Gramm Wasser, 400 Milliliter Ethanol und 20 Milliliter konz. Schwefelsäure wurden 2 Stunden zum Rückfluß (78°C) erwärmt, auf Raumtemperatur abgekühlt, mit Wasser versetzt und mit verdünnter Natronlauge auf pH 3 eingestellt. Anschließend wurde mit Dichlormethan extrahiert, die organische Phase getrocknet, das Lösungsmittel zum größten Teil abgezogen und der Rückstand durch Zugabe von Petrolether zur Kristallisation gebracht. Man erhielt 22 Gramm 4-(5'-Jodpyridyl-2'-oxy)phenol vom Festpunkt 110°C.

## Beispiel 3

a) Analog Beispiel 1a) wird die Umsetzung durchgeführt.

b) 50 Gramm des nach Beispiel 1a) erhaltenen 4-(5'-Nitro-pyridyl-2'-oxy)phenyl-(tert.-butyl)ethers, 770 Milliliter Ethanol, 190 Gramm Wasser und 10 Milliliter konzentrierte Schwefelsäure wurden 4 Stunden bei 50°C gerührt. Die Aufarbeitung analog Beispiel 1b) ergab 30 Gramm 4-(5'-Nitropyridyl-2'-oxy)phenol.

## Vergleichsbeispiel 1

a) Analog Beispiel 1a) wurde die Umsetzung durchgeführt.

b) 50 Gramm des nach Beispiel 1a) erhaltenen 4-(5'-Nitro-pyridyl-2'-oxy)phenyl-(tert.-butyl)ethers, 30 Milliliter 57prozentige Jodwasserstoffsäure und 50 Milliliter Eisessig wurden 4 Stunden bei 100°C gerührt. Die Aufarbeitung ergab hauptsächlich Zersetzungsprodukte.

## Vergleichsbeispiel 2

a) Analog Beispiel 1a) wurde die Umsetzung anstelle des tertiären Butylethers II mit dem Methylether des 4-(5'-Nitropyridyl-2'-oxy)-phenols durchgeführt.

b) 40 Gramm 4-(5'-Nitropyridyl-2'-oxy)phenyl-methylether, 770 Milliliter Ethanol, 190 Milliliter Wasser und 10 Milliliter konz. Schwefelsäure wurden 4 Stunden bei 80°C gerührt. Die Aufarbeitung ergab praktisch quantitativ den Ausgangsstoff.

## Beispiel 4

a) 100 Gramm p-(tert.-Butoxy)-phenol, 200 Milliliter Dioxan, 240 Milliliter Wasser und 112 Gramm Natriumhydroxid wurden bei 80°C gerührt und 80 Gramm Difluorchlormethan eingegast. Das Reaktionsgemisch wurde abgekühlt, mit tert.-Butylmethylether extrahiert, getrocknet, filtriert und eingeengt. Man erhielt 80 Gramm 1-(Difluormethoxy)-4-(tert.-butoxy)-benzol mit einem Siedepunkt von 45—50°C bei 0,1 mbar.

NMR: TMS ($CDCl_3$)
1,30 ppm (5) (9)

6,39 ppm (1) (1)
6,93 ppm (5) (4)

b) 30 Gramm 1-(Difluormethoxy)-4-(tert.-butoxy)-benzol aus Beispiel 4a), 200 Milliliter Ethanol, 50 Milliliter Wasser und 10 Milliliter konz. Schwefelsäure wurden 2 Stunden bei 80°C gerührt. Das Reaktionsgemisch wurde mit Ether extrahiert, die organische Phase getrocknet, filtriert und eingeengt. Man erhielt 16 Gramm 4-Difluormethoxyphenol.

NMR: TMS ($CDCl_3$)
4,12 ppm (S) (1)
6,35 ppm (T) (1)
6,72 ppm (D) (2)
6,95 ppm (D) (2)

**Patentansprüche**

1. Verfahren zur Herstellung der Diether von m- oder p-Hydroxyphenolen der Formel

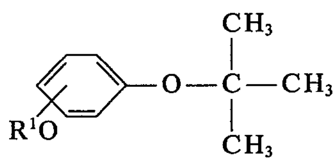

(Ia)

oder Monoether von m- oder p-Hydroxyphenolen der Formel

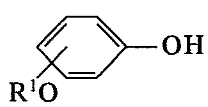

(Ib)

worin die beiden Hydroxygruppen in m- oder p-Stellung zueinander stehen und $R^1$ einen aliphatischen Rest oder den Rest

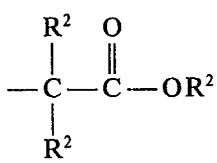

den Rest

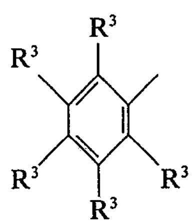

den Rest

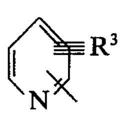

den Rest

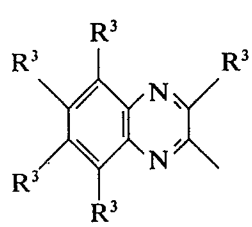

oder den Rest

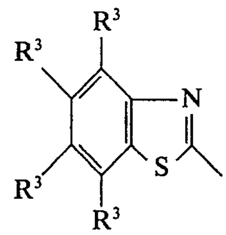

bedeutet, die einzelnen Reste $R^2$ und $R^3$ gleich oder verschieden sein können und jeweils ein Wasserstoffatom, einen aliphatischen, araliphatischen oder aromatischen Rest bezeichnen, $R^3$ auch jeweils für ein Halogenatom, eine Nitrogruppe, Cyangruppe oder eine Alkoxygruppe steht, durch Umsetzung eines Monoethers von Dihydroxyverbindungen mit Halogenverbindungen unter Bildung eines Diethers und Abspaltung der ursprünglichen Monoethergruppe, dadurch gekennzeichnet, daß man

a) m- oder p-tert.-Butoxyphenol der Formel

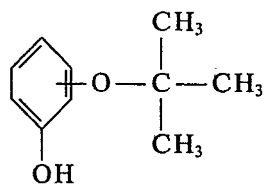

(II)

mit Halogenverbindungen der Formel

$R^1$–X (III)

worin $R^1$ die vorgenannte Bedeutung besitzt und X ein Halogenatom bedeutet, in Gegenwart der Hydroxide, Carbonate und/oder Hydrogencarbonate von Alkalien oder Erdalkalien und von unter den Reaktionsbedingungen inerten organischen Lösungsmitteln bei einer Temperatur von 20 bis 150°C zu den Endstoffen Ia umsetzt und gegebenenfalls

b) die so erhaltenen Endstoffe Ia mit einem Gemisch aus Wasser, wassermischbaren Alkanolen und anorganischen Säuren bei einer Temperatur von 30 bis 100°C zu den Endstoffen Ib umsetzt.

2. Diether von m- oder p-Hydroxyphenol der Formel

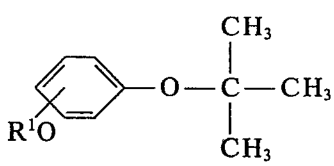

(Ia)

worin die beiden Ethergruppen in m- oder p-Stellung zueinander stehen und $R^1$ einen Halogenalkylrest oder den Rest

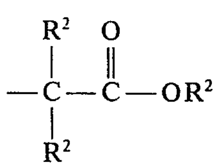

oder den Rest

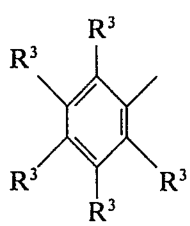

oder den Rest

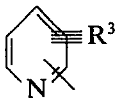

oder den Rest

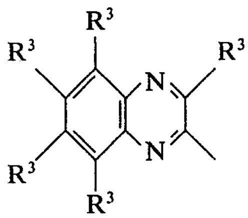

oder den Rest

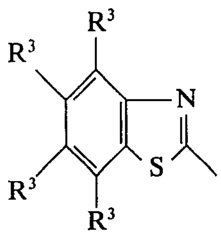

bedeutet, die einzelnen Reste $R^2$ und $R^3$ gleich oder verschieden sein können und jeweils ein Wasserstoffatom, einen aliphatischen, araliphatischen oder aromatischen Rest bezeichnen, $R^3$ auch jeweils für ein Halogenatom, eine Nitrogruppe, eine Cyangruppe oder eine Alkoxygruppe steht.

## Claims

1. A process for the preparation of a diether of m- or p-hydroxyphenol, of the formula

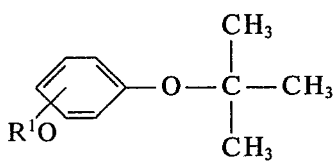

(Ia)

or a monoether of m- or p-hydroxyphenol, of the formula

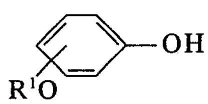

(Ib)

where the two hydroxyl groups are in the m- or p-position relative to one another and $R^1$ is an aliphatic radical or

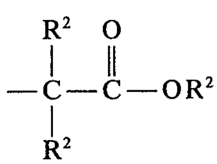

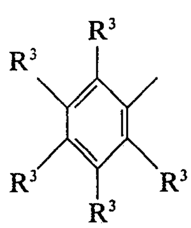

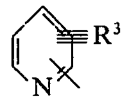

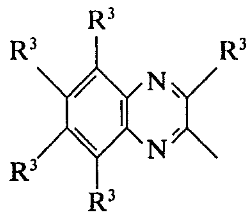

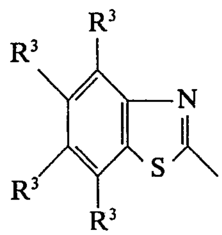

the individual radicals $R^2$ and $R^3$ can be identical or different and each is hydrogen or an aliphatic, araliphatic or aromatic radical, and $R^3$ can also in each case be halogen, nitro, cyano or alkoxy, by reacting a monoether of a dihydroxy compound with a halogen compound to form a diether and splitting off the original monoether group, wherein

a) m- or p-tert.-butoxyphenol of the formula

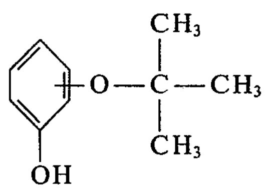

(II)

is reacted with a halogen compound of the formula

$R^1$–X (III)

where $R^1$ has the above meanings and X is halogen, in the presence of a hydroxide, carbonate and/or bicarbonate of an alkali metal or alkaline earth metal and an organic solvent which is inert under the reaction conditions, at from 20 to 150°C, to give the end product Ia, and, if desired,

b) the resulting end product Ia is reacted with a mixture of water, a water-miscible alkanol and an inorganic acid at from 30 to 100°C to give the end product Ib.

2. A diether of m- or p-hydroxyphenyol, of the formula

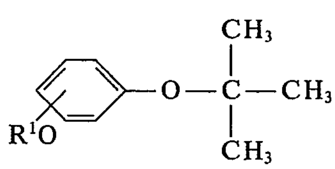

(Ia)

where the two ether groups are in the m- or p-position relative to one another and $R^1$ is haloalkyl or

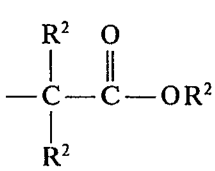

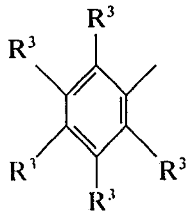

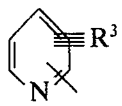

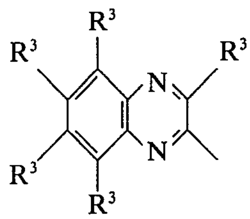

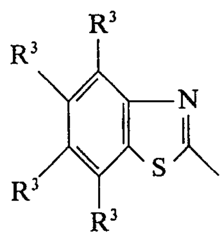

and the individual radicals $R^2$ and $R^3$ can be identical or different and each is hydrogen or an aliphatic, araliphatic or aromatic radical, and $R^3$ can also in each case be halogen, nitro, cyano or alkoxy.

**Revendications**

1. Procédé pour la préparation de diéthers de m- ou p-hydroxyphénols de formule

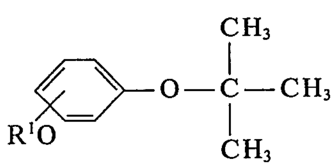

(Ia)

ou de monoéthers de m- ou p-hydroxyphénols de formule

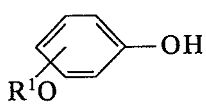

(Ib)

dans lesquelles les deux groupes hydroxy sont en position m ou p l'un par rapport à l'autre et $R^1$ représente un radical aliphatique ou le radical

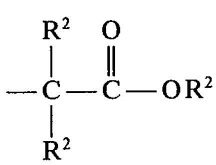

le radical

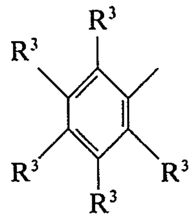

le radical

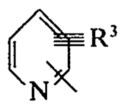

le radical

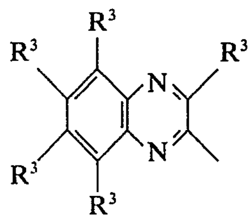

ou le radical

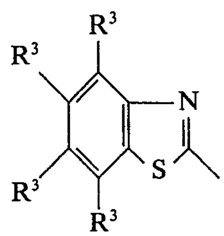

les différents radicaux $R^2$ et $R^3$ pouvant être semblables ou dissemblables et représentant chacun un atome d'hydrogène, un radical aliphatique, araliphatique ou aromatique, $R^3$ pouvant être également mis, dans chaque cas, pour un atome d'halogène, un groupe nitro, un groupe cyano ou un groupe alcoxy, par la mise en réaction d'un monoéther de composés dihydroxylés avec des composés halogénés, avec formation d'un diéther et séparation du groupe monoéther initial, caractérisé en ce que:

a) l'on fait réagir un m- ou p-tertiobutoxyphénol de formule

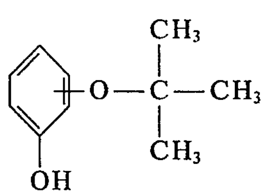

(II)

avec des composés halogénés de formule

$R^1{-}X$ (III)

dans laquelle $R^1$ a la signification donnée ci-dessus et X représente un atome d'halogène, en présence des hydroxydes, carbonates et/ou hydrogénocarbonates de bases alcalines ou alcalino-terreuses et de solvants organiques inertes dans les conditions de la réaction, à une température de 20 à 150°C, pour obtenir les produits ultimes Ia et, le cas échéant,

b) on fait réagir les produits ultimes Ia ainsi obtenus avec un mélange d'eau, d'alcanols miscibles à l'eau et d'acides organiques, à une température de 30 à 100°C, pour obtenir les produits ultimes Ib.

2. Diéthers de m- ou p-hydroxyphénol de formule

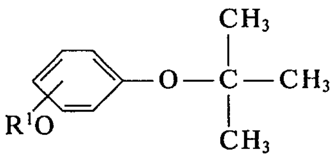

(Ia)

dans laquelle les deux groupes éther sont en position m ou p l'un par rapport à l'autre et $R^1$ représente un radical halogénalkyle ou le radical

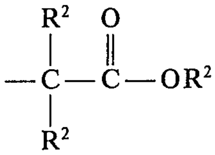

ou le radical

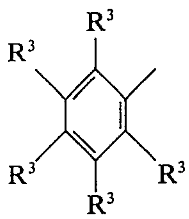

ou le radical

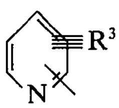

ou le radical

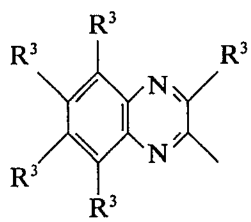

ou le radical

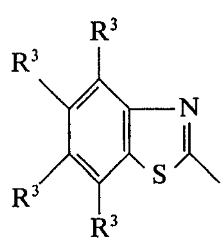

les différents radicaux $R^2$ et $R^3$ pouvant être semblables ou dissemblables et représentent chacun un atome d'hydrogène, un radical aliphatique, araliphatique ou aromatique, $R^3$ pouvant être également mis, dans chaque cas, pour un atome d'halogène, un groupe nitro, un groupe cyano ou un groupe alcoxy.